# EUROPEAN PATENT APPLICATION

(11) **EP 1 947 173 A2**
(43) Date of publication of application: **23.07.2008**
(21) Application number: 08007867.8
(22) Date of filing: 02.09.2003
(51) Int. Cl.: C12N 5/00, C12N 5/06

(54) **Method of preparing expanded primate mammalian blood cells**

(62) Divisional of application: 03818826.4
(71) Applicant: Regenetech, Inc., Sugar Land, TX 77478 (US)
(72) Inventor: Rudd, Donnie, Sugar Land TX 77479 (US)
(74) Representative: Ebner von Eschenbach, Jennifer

(57) **Abstract**

A method of preparing expanded primate mammalian blood adult stem cells is provided, which method comprises the steps of:
a.) placing primate mammalian blood adult stem cells in a rotatable bioreactor; and
b.) controllably expanding the blood adult stem cells by a factor of at least seven times the number per unit volume that were placed in the rotatable bioreactor in less than seven days while maintaining their three-dimensional geometry and their cell-to-cell support and cell-to-cell geometry by rotating the rotatable bioreactor at a speed that suspends the cells to prepare expanded primate mammalian blood cells.

## Description

### Background of the Invention

The present invention relates to a method of repairing primate mammalian tissue.

Regeneration of primate mammalian tissue has long been a desire of the medical community. Thus far, repair of primate mammalian tissue has been accomplished largely by transplantations of like tissue from a donor. Beginning essentially with the kidney transplant from one of the Herrick twins to the other and later made world famous by South African Doctor Christian Barnard's transplant of a heart from Denise Darval to Louis Washkansky on December 3, 1967, tissue transplantation became a widely accepted method of extending life in terminal patients.

Transplantation of mammalian tissue, from its first use, encountered major problems, primarily tissue rejection due to the body's natural immune system. This often caused the use of tissue transplantation to have a limited prolongation of life (Washkansky lived only 18 days past the surgery).

In order to overcome the problem of the body's immune system, numerous anti-rejection drugs (e.g. Imuran, Cyclosporine) were soon developed to suppress the immune system and thus prolong the use of the tissue prior to rejection. However, the rejection problem has continued creating the need for an alternative to tissue transplantation.

Bone marrow transplantation was also used, and is still the procedure of choice for treatment of some illnesses such as leukemia, to repair certain tissues such as bone marrow, but bone marrow transplantation also has problems. It requires a match from a donor (found less than 50% of the time), it is painful, expensive, and risky. Consequently, an alternative to bone marrow transplantation is highly desirable. Transplantation of tissue stem cells such as the transplantation of liver stem cells found in U.S. Patent No. 6,129,911 have similar limitations rendering their widespread use questionable.

In recent years, researchers have experimented with the use of pluripotent embryonic stem cells as an alternative to tissue transplant. The theory behind the use of embryonic stem cells has been that they can theoretically be utilized to regenerate virtually any tissue in the body. The use of embryonic stem cells for tissue regeneration, however, has also encountered problems. Among the more serious of these problems are that transplanted embryonic stem cells have limited controllability, they sometimes grow into tumors, and the human embryonic stem cells that are available for research would be rejected by a patient's immune system (Nature, June 17, 2002). Further, widespread use of embryonic stem cells is so burdened with ethical, moral, and political concerns that its widespread use remains questionable.

It can therefore be seen that a need exists to provide a method of primate mammalian tissue repair not based on organ transplantation or embryonic stem cell utilization.

### Summary of the Invention

The present invention is a method of repairing primate mammalian tissue. The method comprises removing blood cells from the primate mammal, controllably expanding the blood cells by a factor of at least seven times preferably in less than seven days while maintaining their three-dimensional geometry and their cell-to-cell support and cell-to-cell geometry, and reintroducing the expanded blood cells into the primate mammal within a time period sufficient to allow the primate mammal body system to utilize the blood cells to effectively repair damaged tissue.

It is an object of this invention to provide a method of repairing primate mammalian tissue.

It is a further object of this invention to use a combination of expanded blood and the body's ability to repair itself to repair body tissue.

It is still another object of this invention to provide a method of repairing vital body organs without the use of organ transplantation or embryonic stem cell use.

Another object of the present invention is to provide an ex vivo human cell composition having at least eight times the number of blood cells per volume as in the human hematopoietic system from which the cells originated.

And yet another object of this invention is to provide a method of replenishing mammalian cells.

These and still other objects and advantages of the present invention will be apparent from the description of the preferred embodiments that follow.

### Detailed Description of the Invention

The present invention is related to a method of repairing, replenishing and regenerating tissue in primate mammalians.

This invention may be more fully described by the preferred embodiment as hereinafter described, but is not intended to be limited thereto.

In the preferred embodiment of this invention, a method is described to prepare adult stem cells that can assist the body in repairing, replacing and regenerating tissue. Blood cells are removed from a patient. A subpopulation of these cells is currently referred to as adult stem cells. The blood cells are placed in a bioreactor such as that described in United States Patent 5,702,941 and is incorporated herein by reference. The bioreactor vessel is rotated at a speed that provides for suspension of the blood cells to maintain their three-dimensional geometry and their cell-to-cell support and geometry. During the time that the cells are in the reactor, they are fed nutrients, exposed to either hormones, cytokines, or growth factors, and/or genetically modified, and toxic materials are removed. The toxic materials typically removed are from blood cells comprising the toxic granular material of dying cells and the toxic material of granulocytes and mycrophages. A subpopulation of these cells is expanded creating a large amount of cells. The expansion of the cells is controlled so that the cells expand at least seven times in a sufficient amount of time, preferably within seven days. The cells are then injected intravenously or directly into the tissue to be repaired allowing the body's natural system to repair and regenerate the tissue. The method can be used to repair liver tissue, heart tissue, hematopoietic tissue, blood vessels, skin tissue, muscle tissue, gut tissue, pancreatic tissue, central nervous system cells, bone, cartilage, connective tissue, pulmonary tissue, spleen tissue, and other body tissue. Further, in this method blood cells can be manipulated to alter their curative characteristics, preferably by genetically modifying the blood cells.

In still another embodiment of this invention, peripheral blood (PB) cells are obtained from a person needing tissue repair. In brief, mononuclear cells (MNCs) are obtained from the first apheresis product collected from the donors. Prior to apheresis, the donor is treated with G-CSF 6 :g/kg every 12 hr over 3 days and then once on day 4. MNCs are collected by subjecting the donor's total blood volume to 3 rounds of continuous-flow leukapheresis through a separator, such as a Cobe Spectra cell separator.

In another embodiment, the present invention relates to a method of replenishing primate mammalian cells. Blood cells are removed by conventional methods from a primate mammal. The cells are controllably expanded in a method utilizing the biosector described in United States Patent 5,702,441, which is incorporated herein by reference. The cells are expanded while maintaining their three-dimensional geometry and their cell-to- cell geometry. Toxic materials are removed, for example, toxic granular material and mycrophages. The expanded cells are their reintroduced by conventional means into the primate animal within a time period that is sufficient to all the primate mammal body system to effectively replenish different cellular population in the primate mammal.

Another embodiment of the present invention relates to an ex vivo human blood cell composition that functions to assist a body system to repair, replenish and regenerate tissue, for example, the tissue previously described. The blood cell composition has a substantial number of cells originating from a human hematopoietic system that have undergone ex vivo cell division. The composition comprises at least eight times the number of blood cells per volume as in the human hermtopoietic system from which it originated. It has essentially the three-dimensional geometry and the cell-to-cell support and cell-to-cell geometry as the human hermtopoietic system from which it originated. The composition is free of toxic granular material, for example, dying cells and the toxic material or content of granulocytes and mychrophages.

### Operative Method

### A) Collection and maintenance of cells

Collected MNCs (0.75 x 10⁶ cells/ml) obtained from donors as described above are suspended in Iscove's modified Dulbecco's medium (IMDM) (GIBCO, grand Island, NY) supplemented with 20% either fetal calf serum (PCS) (Flow Laboratories, McClean, VA), 5% human albumin (HA) or 20% human plasma, 100 ng/ml recombinant human G-CSF (Amgen Inc., Thousand Oaks, CA), and 100 ng/ml recombinant human stem cell factor (SCF) (Amgen). The culture mix is injected into 300 ml or 500 ml Life Cell nonpyrogenic plastic bags (Baxter, Deerfield, IL) and placed in a bioreactor that is placed in a humidified incubator at 37EC under an atmosphere of 5% CO₂. The culture bags are inspected daily.

### B) Analysis of hermatopoietic colony-forming cells

Hematopoietic colony forming cells are assayed using a modification of a previously described assay. In brief, 10⁵ MNCs are cultured in 0.8% methylcellulose with IMDM, 30% FCS, 1.0 U/ml erythropoietin (Amgen), 50 ng/ml recombinant human GM-CSF (Immunex Corp., Seattle, WA), and 50 ng/ml SCF (Amgen). One-milliliter aliquots of each culture mixture are then placed in 35-mm Petri dishes (Nunc Inc., Naperville, IL) and incubated in duplicate at 37EC in air in a humidified atmosphere of 5% CO₂. All cultures are evaluated after 7 days for the number of burst-forming unit-erythroid (BFU-E) colonies (defined as aggregates of more than 500 hemoglobinized cells or 3 or more erythroid subcolonies), for the number of colony-forming units granulocyte-macrophage (CFU-GM) colonies of granulocytic or monocyte-macrophage cells or both, and for the number of CFU-granulocyte-erythroid macrophage-megakaryocyte (CFU-GEMM) containing all elements. Individual colonies are plucked from the cultures with a micropipette and analyzed for cellular composition.

### C) Analysis of stem cells and lymphocytes

Lymphocytes are analyzed by 2-color staining using the following antibody combinations: CD56+CD16-PE/CD3-FITC, CD3-PE/CD4-FITC, CD3-PE/CD8-FITC, CD19-PE. Controls include IgG1-PE/IgG1-FITC for isotype and CD14-PE/CD45-FITC for gating. Progenitor cells are analyzed by 3-color staining with the fluorochromes PerCP/PE/FITC using the following antibody combinations: CD45/CD90/CD34, CD45/CD34/CD38, CD45/CD34/CD33, and CD45/CD34/CD15. CD45/IgG1/IgG1 is used as a control. In brief, 10⁶ cells from the donor are incubated with 10:1 of antibodies at 2-8EC for 15 minutes in the dark and then washed twice in phosphate-buffered saline. Then the cells are resuspended, fixed with 1% formaldehyde, and analyzed on a FACScan flow cytometer (Becton-Dickinson) equipped with CELLQuest software (Becton Dickinson). For analyses of lymphocytes, 10,000 cells are acquired from each tube, and then gated on the basis of the forward and right angle light scatter patterns. The cutoff point is visually set at a level above background positivity exhibited by isotype controls. For analyses of progenitor cells, 75,000 cells from each tube is acquired and then sequentially gated.

### D) Increase in amount of hermatopoietic colony-forming cells

Incubation of the donors' PB cells in this tissue culture system significantly increases the numbers of hematopoietic colony-forming cells. A constant increase in the numbers of CFU-GM (up to 7-fold) and CFU-GEMM (up to 9-fold) colony-forming cells is observed up to day 7 with no clear plateau.

### E) Increase in CD34+ cells

Incubation of MNCs from normal donors in this tissue culture system significantly increases the numbers of CD34+ cells. The average number of CD34+ cells increased 10-fold by day 6 of culture and plateaus on that same day. The relative number of CD34+ cells co-expressing the myeloid-lineage markers CD15 and CD33 increases significantly by days 5 and 6. After the seventh day, the cells are reinjected into the patient. The injection can be an injection of the cells into the bloodstream or, prererably, an injection directly into the injured tissue such as the liver.

Features of the parent application include:
1. A method of repairing primate mammalian tissue comprising removing blood cells from the primate mammal, controllably expanding the blood cells by a factor of at least seven times in less than seven days while maintaining their three- dimensional geometry and their cell-to-cell support and cell-to-cell geometry, and reintroducing the expanded blood cells into the primate mammal within a time period sufficient to allow the primate mammal body system to utilize the blood cells to effectively repair damaged tissue.
2. A method as in Feature 1 wherein the expanded blood cells have any toxic material therein removed prior toreintroduction into the primate mammal body.
3. A method as in Feature 1 wherein the removing of any toxic materials from the blood cells comprises removing the toxic granular content of dying cells and the toxic content of granulocytes and mycrophages.
4. A method as in Feature 1 wherein the primate mammalian tissue being repaired is a vital organ.
5. A method as in Feature 1 wherein the primate mammal is a human.
6. A method as in Feature 1 wherein the primate mammalian tissue being repaired is heart tissue.
7. A method as in Feature 1 wherein the tissue being repaired is liver tissue.
8. A method as in Feature 1 wherein the tissue being repaired is hematopoietic tissue.
9. A method as in Feature 1 wherein the tissue being repaired is blood vessels.
10. A method as in Feature 1 wherein the tissue being repaired is skin tissue.
11. A method as in Feature 1 wherein the tissue being repaired is muscle tissue.
12. A method as in Feature 1 wherein the tissue being repaired is gut tissue.
13. A method as in Feature 1 wherein the tissue being repaired is pancreatic tissue.
14. A method as in Feature 1 wherein the tissue being repaired is central nervous system cells.
15. A method as in Feature 1 wherein the tissue being repaired is bone.
16. A method as in Feature 1 wherein the tissue being repaired is cartilage tissue.
17. A method as in Feature 1 wherein the tissue being repaired is connective tissue.
18. A method as in Feature 1 wherein the tissue being repaired is pulmonary cells.
19. A method as in Feature 1 wherein the tissue being repaired is spleen tissue.
20. A method as in Feature1, which includes manipulating the blood cells to alter their curative characteristics.
21. A method as in Feature 1 wherein the manipulating of the blood cells includes genetically modifying the blood cells.
22. A method of replenishing primate mammalian cells comprising removing blood cells from the primate mammal, controllably expanding the blood cells while maintaining their three-dimensional geometry and their cell-to-cell support and cell-to-cell geometry, removing any toxic materials from the blood cells, and reintroducing the expanded blood cells into the primate mammal within a time period sufficient to allow the primate mammal body systems to effectively replenish different cellular populations.
23. An ex vivo human blood cell composition in which a substantial number of cells originating from a human hematopoietic system have undergone ex vivo cell division, comprising a composition having at least eight times the number of blood cells per volume as in the human hematopoietic system from which it originated and having essentially the three-dimensional geometry and the cell-to-cell support and cell-to-cell geometry as the human hematopoietic system from which it originated, said composition being free of toxic granular content of dying cells and the toxic content of granulocytes and mycrophages.
24. An ex vivo human blood cell composition as in Feature 23 containing recombinant human G-CSF.

## Claims

1. A method of preparing expanded primate mammalian blood adult stem cells **characterized by** the combination of steps comprising:
a. placing primate mammalian blood adult stem cells in a rotatable bioreactor; and
b. controllably expanding the blood adult stem cells by a factor of at least seven times the number per unit volume that were placed in the rotatable bioreactor in less than seven days while maintaining their three- dimensional geometry and their cell-to-cell support and cell-to-cell geometry by rotating the rotatable bioreactor at a speed that suspends the cells to prepare expanded primate mammalian blood cells.

2. The method as in claim 1 wherein the expanded blood adult stem cells have any toxic material therein removed.

3. The method as in Claim 2 wherein the removing of any toxic materials from the adult stem cells comprises removing the toxic granular content of dying cells and the toxic content of granulocytes and macrophages.

4. The method as in Claim wherein the primate mammal is a human.

5. A method as in Claim 1, which includes manipulating the blood adult stem cells to alter their curative characteristics.

6. A method as in Claim 1 wherein the manipulating of the blood adult stem cells includes genetically modifying the blood cells.
